Europäisches Patentamt

European Patent Office

Office européen des brevets

(19)

(11) Publication number:

**0 359 952**

**A2**

(12) **EUROPEAN PATENT APPLICATION**

(21) Application number: 89114006.3

(51) Int. Cl.⁵: **B01J 8/04 , C07C 29/15**

(22) Date of filing: 28.07.89

(30) Priority: 05.08.88 CH 2967/88

(43) Date of publication of application:
28.03.90 Bulletin 90/13

(84) Designated Contracting States:
**AT DE ES FR GB GR IT NL**

(71) Applicant: AMMONIA CASALE S.A.
Via della Posta 4
CH-6900 Lugano(CH)

Applicant: Zardi, Umberto
Via Lucino 57
CH-6932 Breganzona (Ti)(CH)

(72) Inventor: Zardi, Umberto
Via Lucino 57
CH-6932 Breganzona(CH)
Inventor: Pagani, Giorgio
Salita dei Frati 13
CH-6900 Lugano(CH)

(74) Representative: Incollingo, Italo
AMMONIA CASALE S.A. Via della Posta 4
CH-6900 Lugano(CH)

(54) **System for the improvement of reactors for the synthesis of methanol and reactors obtained therefrom.**

(57) With the system for the improvement in situ of conventional reactors for the synthesis of methanol the catalytic mass is divided into several beds in series, each bed having a bottom and a conical diaphragm spaced from the free surface of the next catalytic bed in such a way as to create a space to the outer periphery of which the quench gas is fed so as to achieve in said space the optimum mixing with the partially reacted gas which has run axially through the upper catalytic bed, a tube is introduced below and central to the upper catalytic bed delimiting internally the catalytic mass of the lower beds from the upper bed; and the lower bed or beds with maximum pressure drop are transformed into a bed (s) with a substantially radial flow by introducing two cylindrical walls coaxial with said tube substantially perforated and forming airspaces with the shell inner wall and with the tube outer wall respectively.

## System for the improvement of reactors for the synthesis of methanol and reactors obtained therefrom.

This invention concerns a system for the improvement of a reactor for the heterogeneous synthesis of methanol (from gaseous carbon monoxide and hydrogen), in the presence of a granular catalyst contained in a pressure-resistant cylindrical body, the hot reacted gas being mixed with fresh quench gas.

Various processes and reactors for the synthesis of methanol are well known; one of the best established is the ICI process the fundamental characteristics of which are the use of low synthesis pressure (for example 40 to 50 bar) and a copper-based catalyst.

The reactor to operate the process under consideration is in several stages and the temperature of the reacted gas coming from the stages preceding the last stage is controlled by mixing it with quench gas.

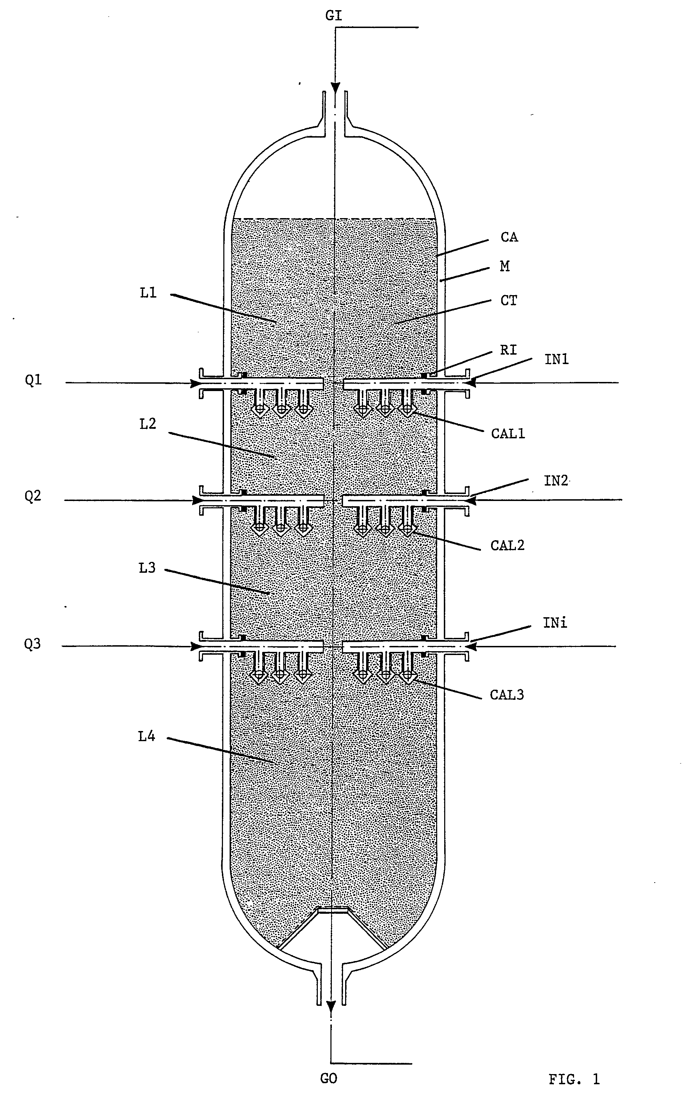

In order to fix ideas properly and straight away, fig. 1 shows a longitudinal cross-section of the conventional methanol reactor consisting of a pressure-resistant shell M with internal surface CA and containing catalyst CT in several layers, for example in four layers or beds L1, L2, L3 and L4 and three feeds of quench gas Q1, Q2, Q3 substantially on the bottom of beds L1, L2, L3.

In effect mixing of reaction gas coming from GI and partially reacted on a bed for example L1 with the respective quench gas Q1 (and so on for gas from L2 with Q2 etc. etc.) takes place in special diamond-shaped chambers CAL1, CAL2, CAL3 created inside the catalytic mass.

Since these chambers represent a line of least resistance to the passage of gas compared to the adjacent zone accupied by the catalyst it follows that the gas flows by preference in said chambers CAL1, CAL2, CAL3 where it can be easily mixed and cooled by the quench gas.

The structure of a well-established conventional reactor of the type shown in Fig. 1 is therefore remarkably simple (for example it does not require intermediate bottoms) and has a high degree of packing.

Nevertheless, such a structure suffers from a number of drawbacks of which we shall just mention:
- difficulty in obtaining an even quench temperature, since the flow of gas into the lozenge-shaped chambers can be influenced by the state of the catalyst immediately in contact with the grids and can therefore vary from chamber to chamber;
- a remarkable degree of mechanical stress on the catalyst contained in the lower layers on which the entire weight of the catalyst is unloaded;

- high pressure drop due to the exclusively axial flow of the gas.

The object of this invention is to provide a methanol reactor without the foregoing drawbacks and this is obtained by making some critical but simple alterations in situ to the conventional reactor. It has surprisingly been found in effect that, while keeping the same shell, it is possible to improve substantially yields and reduce energy consumption, operating for example as follows: the catalytic mass is divided into several beds in series each one having a bottom and a conical diaphragm spaced from the free surface of the following bed in such a way as to obtain a space, to the outer edge of which the quench gas is fed so as to achieve in said space the optimal mixing with the partially reacted gas which has run axially through the upper catalytic bed, a tube is introduced below and centrally to the upper catalytic bed to delimit internally the catalytic mass of the beds below the upper bed; and the lower bed or beds with maximum pressure drop are transformed into a bed (s) with substantially radial flow by introducing two cylindrical walls coaxial with said tube substantially perforated and forming airspaces with the shell inner wall and correspondingly with the tube outer wall.

Reactors are thus obtained with a structure as described in claim 5.

The various aspects and advantages of the invention will be better illustrated by the following description of a preferred but not limitative embodiment.

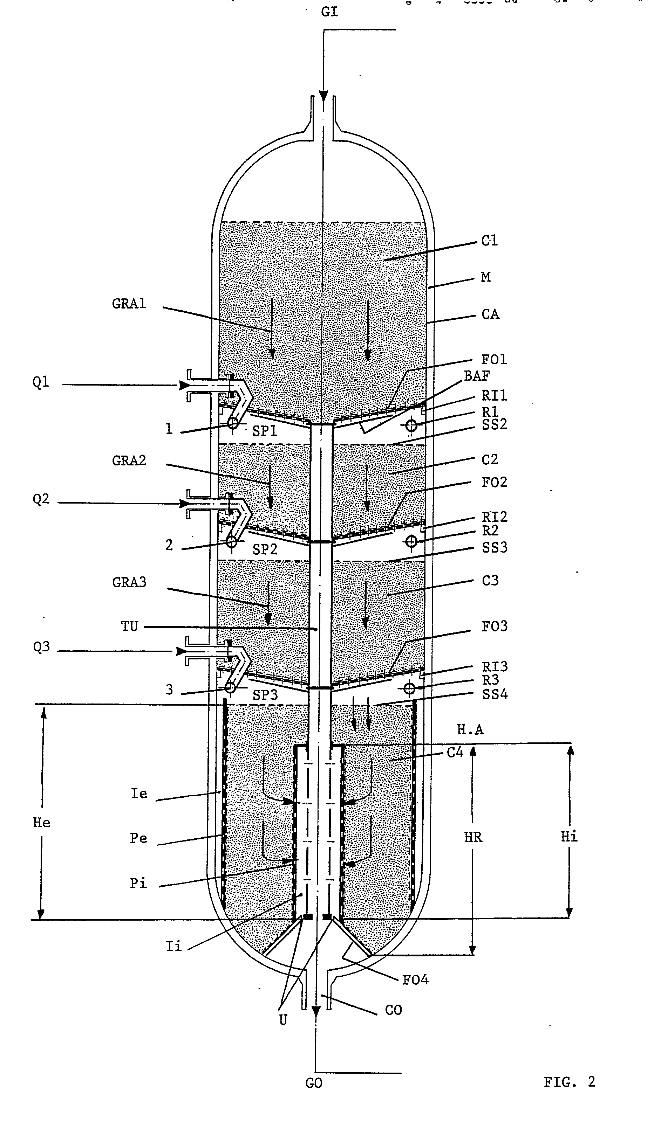

Fig. 2 shows a reactor obtained with the system of modification in situ according to the invention. As can be seen, the system consists of the following measures:

1) The catalytic layers are divided by inserting preferably conical bottoms FO1, FO2, FO3 ... FOi ... FOn-1 at the base of the beds; critically each bottom is spaced from the free surface SS2, SS3, SS4 ... SSi in order to create anular spaces SP1, SP2 ... SPi such as to permit the optimal mixing of the partially reacted gas GRA1 ... GRAi flowing in axially from beds C1 ... Ci, and the quench gas Q1 ... Qi ... Qn-1 introduced substantially from inlets IN1 ... INi in the reactor to be improved.

2) The lozenge-shaped quench chambers CAL1, CAL2 ... CALi are replaced by said spaces SP1 ... SPi between the perforated conical bottoms FOi of the upper beds and the free surface SSi of the lower beds, but the quench gas is now introduced in such a way as to come out close to the inner wall CA of shell M; in other words, ducts 1, 2, 3, etc. feeding quench gas Q1 - Q3 have their outlets at the furthest edge from the reactor central axis, for example as described in a previous patent application by the Applicants.

3) A tube TU which runs through all the catalytic baskets from C2 to Cn (all, that is to say, except the top basket C1) is placed below and in the middle of bottom FO1 of the upper bed; said tube TU delimits internally the catalyst in baskets from C2 to Cn-1 along their entire length, and the catalyst in Cn (C4) from H to A.

4) At least the last bed, i.e. the lower bed or beds where maximum pressure drop is experienced, is converted from a bed with totally axial flow to a bed with substantially radial flow, and more particularly axial-radial; to that end, in bed C4 (or generally Cn) are introduced: a substantially perforated external cylindrical wall Pe with diameter De, which forms airspace le with internal wall CA; an internal cylindrical wall Pi with diameter Di with height Hi < He forming airspace Ii with the outer wall of tube TU; a conical bottom FO4 extending between cartridge CA and the inside of airspace Ii.

As in previous patents by the Applicants (for example US patents No. 4,372,920 and No. 4,755,362) on the difference in height H.A = He - Hi the flow will be axial and along height HR the flow will be radial.

The totally reacted gas leaves from outlet GO.

In an advantageous embodiment conical bottoms FOi consist of several elements going through the reactor manhole, these elements being segments of perforated plate with welded net in contact with the catalyst, supported by beams which at one end rest on central tube TU and at the other end on a ring RI1 originally provided in the reactor shell.

In Fig. 2 the first three beds remain axial while the last bed is turned into an axial radial bed.

The gas flowing axially in the first three beds is sent from the inside towards the outside of the bed by means of diaphragms BAF and quenching takes place close to the shell internal wall CA by means of quench ring R1. Full mixing of the two gas streams is favoured by the impact between quench gas flowing inwardly and the partially reacted gas coming from the catalytic bed with an outward flow.

According to a remarkable aspect of the invention, the presence of a certain number of fins placed on the diaphragms, rotated by 45° from the radius and welded to the above-mentioned conveyor diaphragms, imparts to the partially reacted gas a rotary movement which facilitates the perfect mixing of the two gas components and the achieving of a uniform temperature of the gas entering the next bed.

Only in the last bed is the flow substantially radial, and more particularly axial-radial, thanks to two collectors creating an airspace, one outside the bed (inlet collector) and the other inside.

It should be noted that the greatest pressure drop in these reactors takes place in the 4th bed both because it receives the greatest flow and because the height of the catalyst is greater. By making this 4th bed radial it is therefore possible to achieve a noticeable reduction in pressure drop as compared with the axial flow, while maintaining the typical semplicity of the reactor.

It is possible, however, further to increase reactor performance by converting also the other beds from axial to axial-radial, even if this affects the semplicity of the reactor and increases investment costs.

By modifying the reactor in situ according to the invention the following advantages can be obtained:
- very efficient quenching with easy control of the temperature;
- independent catalytic beds with specific pressures contained within the catalyst;
- reduced pressure drop.

The invention has been described with reference to the preferred embodiment shown in the drawings, with four catalytic beds, the first three with axial flow, the fourth converted to a substantially radial flow, and three quench gas feeds. Obviously the modification in situ according to the invention can be applied to any conventional axial reactor, with a number of beds other than four, with at least one radial or radial-axial bed, and with quench gas feeds in greater or smaller number than three.

## Claims

1. System for the improvement of reactors for the heterogeneous synthesis of methanol consisting of an outer shell and a layered granular catalyst, and of the means of feeding the quench gas inside the catalytic layers, characterized by the fact that the catalytic mass is divided into several beds in series, each bed having a bottom and a conical diaphragm spaced away from the free surface of the next catalytic bed in such a way as to create a space, to the outer periphery of which the quench gas is fed so as to achieve in said space the optimal mixing with the partially reacted gas which has run axially through the upper catalytic bed, a tube is introduced below and central to the upper catalytic bed to delimit internally the catalytic mass of the lower beds from the upper bed; and the lower bed or beds with maximum pressure drop are transformed into a bed (s) with substantially radial flow by introducing two cylindrical walls coaxial with said tube substantially perforated and forming airspaces with the shell inner wall and with the tube outer wall respectively.

2. System according to claim 1, characterized by the fact that the quench gas fed to the outer periphery of the mixing space flows in a centripetal direction and is mixed with the partially reacted gas flowing axially from the bottom of each catalytic bed which is directed centrifugally for example from diaphragms situated under the bottom.

3. System according to claim 2, characterized by the fact that said partially reacted gas is given a rotatory movement, for example thanks to fins situated on the diaphragms and rotated by 45° from the radius.

4. System substantially according to the above descriptions and representations.

5. Reactor for the synthesis of methanol, obtained with the system of improvement in situ according to the foregoing claims, characterized by: a plurality of beds with axial flow separated one from the other by a mixing space to the outer edge of which is ged the quench gas; at least one lower bed with radial or axial radial flow; a central tube going through the axial flow beds below the upper bed, and the bed (s) with substantially radial flow; conical perforated bottoms below each axial flow bed and diaphragms with rotated fins below each perforated bottom; two cylindrical walls substantially perforated forming airspaces with the shell inner wall and with said tube outer wall respectively, and a conical bottom below the last substantially radial bed.

6. Reactor substantially according to the above descriptions and claims.

FIG. 1

FIG. 2